# EUROPÄISCHE PATENTSCHRIFT

(11) **EP 1 117 417 B1**
(45) Veröffentlichungstag und Bekanntmachung des Hinweises auf die Patenterteilung: **12.11.2003**
(21) Anmeldenummer: 99948828.1
(22) Anmeldetag: 22.09.1999
(51) Int. Cl.: A61K 35/78, A61P 25/06

(54) **PHARMAZEUTISCH WIRKSAMES, PFLANZLICHES PRÄPARAT ZUR BEHANDLUNG VON MIGRÄNE**
PHARMACEUTICALLY ACTIVE PLANT PREPARATION FOR THE TREATMENT OF MIGRAINE
PREPARATION VEGETALE D'ACTION PHARMACEUTIQUE DESTINEE AU TRAITEMENT DE LA MIGRAINE

(30) Priorität: 30.09.1998 DE 19844836
(43) Veröffentlichungstag der Anmeldung: 25.07.2001
(73) Patentinhaber: HEXAL AG, D-83607 Holzkirchen (DE)
(72) Erfinder: SPIESS, Stefan, D-83666 Schaftlach (DE)
(86) Internationale Anmeldenummer: EP9907045
(87) Internationale Veröffentlichungsnummer: WO00018415

(56) Entgegenhaltungen:
- WO-A-98/39018
- ROBIN J. MARLES ET AL.: "A BIOASSAY FOR INHIBITION OF SEROTONIN RELEASE FROM BOVINE PLATELETS." JOURNAL OF NATURAL PRODUCTS., Bd. 55, Nr. 8, August 1992 (1992-08), Seiten 1044-1056, XP000867035 XX, XX ISSN: 0163-3864

## Beschreibung

Die Erfindung betrifft ein pharmazeutisch wirksames, pflanzliches Präparat mit verbesserter Wirkung zur ganzheitlichen Behandlung von Migräne, welches pflanzliche Bestandteile und/oder Zubereitungen von Tanacetum parthenium in Kombination mit Vitex agnus castus und/oder Cimicifuga racemosa und/ oder Zingiber officinale als pharmazeutisch wirksame Komponenten enthält.

Die Migräne wird heute als komplexe Funktionsstörung neuronaler und vaskulärer Elemente des ZNS verstanden. Die damit verbundenen Kopfschmerzen werden wahrscheinlich durch eine aseptische Entzündung der Blut- und Duragefäße des Gehirns ausgelöst, verbunden mit einer Durchlässigkeit der Gefäßwände für Albumin und einer Freisetzung von Neurotransmittern wie Serotonin und Tryptamin. Dabei verläuft das Krankheitsgeschehen in der Reihenfolge Vasodilation, Trigeminus-Aktivierung, neurogene Entzündung. Die Migräneattacken treten anfallartig und wiederholt auf. Dabei handelt es sich um halbseitig auftretende Kopfschmerzen, die mit verschiedenen Begleiterscheinungen einhergehen können: vegetative Symptome wie Übelkeit und Erbrechen, Licht- und Lärmscheu, visuelle Symptome wie Sehstörungen, außerdem neurologische Ausfälle wie Lähmungserscheinungen oder Sprach- und Sprechstörungen. Die Symptome Übelkeit und Erbrechen haben ihre Ursache in der zum Erliegen gekommenen Magen- und Darmperistaltik. Den Hauptanteil (70%) der Migränepatienten stellen Frauen dar. Diese weisen eine verstärkte Migränehäufigkeit in Zusammenhang mit den Regelblutungen auf. Die Migränebehandlung beschränkt sich bisher darauf, die Kopfschmerzen zu behandeln und somit eine Erleichterung für den Patienten zu erlangen. Eine effektivere und verbesserte Behandlung der Migräne mittels Phytopharmaka, indem auch die anderen Migränesymptome angesprochen werden, ist nicht bekannt.

Ein traditionell zur Behandlung von Migräne verwendetes Phytopharmakon stellt Tanacetum parthenium (Feverfew), zur Familie der Asteraceae gehörend, dar. Das blühende Kraut, aber besonders die Blätter der Tanacetum parthenium der englischen Wuchsform, enthalten neben ätherischen Öl, Kampfer, Borneol und Pinen vor allem Sesquiterpenlactone. Als Sesquiterpenlactone wurden Michefuscalid, cis-Chrysanthenylacetat und das mengenmäßig vorherrschende Parthenolid, das damit als Leitsubstanz gelten kann, isoliert. Bei der Migräne muß eine medikamentöse Therapie sinnvoll sein, die die Parameter Gefäßstörung, Schmerz und Entzündung beeinflussen kann. Die Sesquiterpenlactone, insbesondere Parthenolid, bewirken eine antiphlogistische Wirkung durch Hemmung der Prostaglandinsythese, verbessern die Fließeigenschaften des Blutes durch Hemmung der Thrombozytenaggregation, reduzieren die Serotoninfreisetzung, hemmen die Histaminfreigabe und weisen spasmolytische Wirkung auf. Hauptsächlich wird Tanacetum parthenium zur Prophylaxe verabreicht. Die für die Migräne typische psychischen und physischen Symptome werden durch die Einnahme von Tanacetum parthenium gemildert, aber häufig beeinträchtigen sie die betroffenen Personen immer noch ganz beträchtlich, so daß die Lebensqualität wesentlich eingeschränkt ist.

Die in der Migränetherapie gängigen nicht pflanzlichen Wirkstoffe wie Sumatriptan, Ibuprofen, Acetylsalicylsäure, etc. haben ebenso das Problem, daß häufig kein ausreichender Therapieerfolg zu verzeichnen ist, wobei noch zusätzlich die Gefahr besteht, daß ganz erheblichen Nebenwirkungen auftreten.

WO 98/39018 offenbart die Kombination von Tanacetum parthenium mit Salix alba zur Behandlung der Migräne.

"A bioassay for inhibition of serotonin release from bovine platelets" (R. J. Marles et al., Journal of natural products , Bd. 55, 1992, S. 1044-1056) beschreibt einen in-vitro Test, der auf der Inhibierung der Serotonin-Freisetzung durch bestimmte Substanzen beruht. Einzeln getestet wurden Tanacetum parthenium, Artemisia absinthium und Zingiber officiale, Stoffe die in der Migränetherapie verwendet werden.

Die Aufgabe der vorliegenden Erfindung ist es nun, ein in der Wirksamkeit verbessertes, effektiveres Phytopharmakon zur Behandlung von Migräne bereitzustellen, welches keine oder nur geringfügige Nebenwirkungen zur Folge hat.

Es wurde nun überraschenderweise gefunden, daß die erfindungsgemäße Aufgabe durch eine Kombination von Tanacetum parthenium mit anderen Arzneipflanzen, wie beispielsweise Vitex agnus castus und/ oder Cimicifuga racemosa und/ oder Zingiber officinale (Ingwer), gelöst werden kann. Die Kombination von Tanacetum parthenium mit Zingiber officinale führt zu einer deutlich verbesserten Wirkung, wobei neben der Linderung der Kopfschmerzen zudem auch die übrigen Migränesymptome, insbesondere Schwindel, Übelkeit und gastrointestinale Beschwerden minimiert werden können. Eine Kombination von Tanacetum parthenium mit Vitex agnus castus bzw. Cimicifuga racemosa sowie die Dreierkombination von Tanacetum parthenium, Zingiber officinale und Vitex agnus castus führt insbesondere bei Frauen, deren Migräneanfälle schwerpunktmäßig in Zusammenhang mit den Regelblutungen auftreten, zu einer deutlichen Wirkungsverstärkung bzw. Linderung aller Migränesymptome.

Vitex agnus castus (Mönchspfeffer, Keuschlamm) gehört zu der Familie der Verbenaceen (Eisenkrautgewächse). Die Früchte stellen den genutzten Pflanzenbestandteil dar. Als Inhaltsstoffe wurden verschiedene sekundäre Pflanzenstoffe nachgewiesen, und zwar Iridoide, Flavonoide und etherische Öle. Die Fähigkeit dieser Stoffe, gezielt an laktotropen Zellen anzugreifen und sich dort an Dopamin-Rezeptoren zu binden, erklärt die gute Wirksamkeit beim prämenstruellen Syndrom. Unter dem Begriff prämenstruelles Syndrom versteht man eine wiederkehrende physische und psychische Störungen und/ oder Änderungen des Verhaltens, die regelmäßig nur in der Gelbkörperphase des Menstruaktionszyklus auftreten können. Die mit diesem Krankheitsbild verbundene verstärkte Prolaktinausschüttung wird durch dieses Phytopharmakon deutlich reduziert. Eine zu hohe Prolaktinkonzentration im Blut vermindert die pulsatorische Gonatropin-Sekretion, die letztlich entscheidend für einen normalen Menstruationszyklus ist.

Cimicifuga racemosa (Traubensilberkerze) gehört zu der Familie der Hahnenfußgewächse (Ranunculaceae). Pharmazeutisch genutzt wird der Wurzelstock. Die wertbestimmenden Inhaltsstoffe sind die Triterpenglykoside, insbesondere die Xyloside Actein und Cimifugosid. Die Zubereitung der Droge besitzt hormonartige Eigenschaften, wobei die östrogene Wirkkomponente besonders dominiert. Diese Drogenzubereitungen werden üblicherweise zur Behandlung des prämenstruellen Syndroms und klimakterischer Symptome verwendet.

Zingiber officinale (Ingwer) wird neben der weltweiten Bedeutung als Gewürz und Grundstoff für die Lebensmittelindustrie seit mehreren Jahrhunderten als Arzneimittel genutzt. Die pharmazeutisch genutzte Wurzel von Zingiber officinale enthält bis zu 3% ätherisches Öl (Ingweröl), das als quantitative Hauptbestandteile Sesquiterpenkohlenwasserstoffe und Sesquiterpenalkohole, vor allem Zingiberen (30%) und β-Bisabolen (10-15%) besitzt. Außerdem enthält sie diverse Scharfstoffe wie z.B. Gingerole und Shogaole, welche eine hohe therapeutische Wirksamkeit aufweisen. Zingiber officinale wird in der modernen westlichen Medizin hauptsächlich in Form von Pulvern, Extrakten, Destillaten, Aufgüssen, Tinkturen und als ätherisches Zingiber officinale Öl verwendet. Er wird zur Verhütung der Symptome der Reisekrankheit, aber auch ganz allgemein als Antiemetikum eingesetzt. Darüber hinaus wird Zingiber officinale als Karminativum, Spasmolytikum, Antiflatulens, Digestivum, Aperitivum, Stomachikum, Expektorans, Antitussivum, Adstringens, Stimulans und Tonikum verwendet.

Unter den pflanzlichen Bestandteilen werden hier die pharmazeutisch genutzten Pflanzenteile der wirksamen Komponenten, wie z.B. Blätter, Früchte, Wurzelstock, sowie deren getrockneten Formen verstanden.

Die pflanzlichen Zubereitungen können in der Form von Extrakten, Pulvern, Destillaten, Aufgüssen, Tinkturen und Ölen vorliegen.

Das erfindungsgemäße pflanzliche Präparat kann in der Form von Kapseln, Filmtabletten, Lösungen, Dragees, Suppositorien, Brausetabletten, Kautabletten oder Brausegranulat vorliegen.

Die in dem erfindungsgemäßen pflanzlichen Präparat eingesetzte Menge an pflanzlichen Bestandteilen bzw. Zubereitung von Tanacetum parthenium wird derart gewählt, daß sie einer Menge von 0,1- 1 mg, insbesondere 0,2-0,6 mg, Parthenolid entspricht.

Die in dem erfindungsgemäßen pflanzlichen Präparat eingesetzte Menge an pflanzlichen Bestandteilen bzw. an einer Zubereitung von Cimicifuga racemosa beträgt 20 bis 100 mg.

Die in dem erfindungsgemäßen pflanzlichen Präparat eingesetzte Menge an pflanzlichen Bestandteilen bzw. an einer Zubereitung von Vitex agnus castus beträgt 20 bis 100 mg, wobei vorzugsweise eine Menge von 20 bis 40 mg verwendet wird.

Die in dem erfindungsgemäßen pflanzlichen Präparat eingesetzte Menge an pflanzlichen Bestandteilen bzw. an einer Zubereitung von Zingiber officinale beträgt 0,5 bis 6 g, wobei vorzugsweise eine Menge von 1bis 4g verwendet wird.

Die Verwendung des erfindungsgemäßen pflanzlichen Präparates, welches pflanzliche Bestandteile und/ oder Zubereitungen von Tanacetum parthenium in Kombination mit weiteren pflanzlichen Komponenten ausgewählt aus der Gruppe von Vitex agnus castus und/oder Cimicifuga racemosa und/ oder Zingiber officinale enthält, erfolgt zur Behandlung oder Prophylaxe von Migräne, insbesondere bei Frauen in Zusammenhang mit den Regelblutungen bzw. menstruellen Beschwerden oder zusätzlichen gastrointestinalen Beschwerden.

Tanacetum parthenium kombiniert mit Vitex agnus castus, Tanacetum parthenium kombiniert mit Cimicifuga racemosa, Tanacetum parthenium kombiniert mit Zingiber officinale und Tanacetum parthenium kombiniert mit Vitex agnus castus und Zingiber officinale stellen die bevorzugten erfindungsgemäßen Kombinationspräparate hinsichtlich der oben beschriebenen Verwendung dar.

## Patentansprüche

1. Pflanzliches pharmazeutisches Präparat zur Behandlung von Migräne, **gekennzeichnet durch** die Kombination von Tanacetum parthenium mit weiteren pflanzlichen Komponenten ausgewählt aus der Gruppe von Vitex agnus castus und/ oder Cimicifuga racemosa und/ oder Zingiber officinale.

2. Pflanzliches pharmazeutisches Präparat zur Behandlung von Migräne nach Anspruch 1, **gekennzeichnet durch** die Verwendung von Bestandteilen und/ oder Zubereitungen der pharmazeutisch wirksamen Komponenten.

3. Pflanzliches pharmazeutisches Präparat zur Behandlung von Migräne nach Anspruch 1 oder 2, **gekennzeichnet durch** Zubereitungen in der Form von Extrakten, Pulvern, Destillaten, Aufgüssen, Tinkturen und Ölen.

4. Pflanzliches pharmazeutisches Präparat zur Behandlung von Migräne nach einem der vorangegangenen Ansprüche, **gekennzeichnet durch** die Kombination von Tanacetum parthenium mit Vitex agnus castus.

5. Pflanzliches pharmazeutisches Präparat zur Behandlung von Migräne nach einem der vorangegangenen Ansprüche, **gekennzeichnet durch** die Kombination von Tanacetum parthenium mit Cimicifuga racemosa.

6. Pflanzliches pharmazeutisches Präparat zur Behandlung von Migräne nach einem der vorangegangenen Ansprüche, **gekennzeichnet durch** die Kombination von Tanacetum parthenium mit Vitex agnus castus und Zingiber officinale.

7. Pflanzliches pharmazeutisches Präparat zur Behandlung von Migräne nach einem der vorangegangenen Ansprüche, **gekennzeichnet durch** die Kombination von Tanacetum parthenium mit Zingiber officinale.

8. Pflanzliches pharmazeutisches Präparat zur Behandlung von Migräne nach einem der vorangegangenen Ansprüche, **gekennzeichnet durch** Kapseln, Filmtabletten, Lösungen, Dragees, Suppositorien, Brausetabletten, Kautabletten und Brausegranulat als Darreichungsform.

9. Pflanzliches pharmazeutisches Präparat zur Behandlung von Migräne nach einem der vorangegangenen Ansprüche, **gekennzeichnet durch** einen Gehalt an Tanacetum parthenium in Form der pflanzlichen Bestandteile und/ oder einer Zubereitung, die 0,1- 1 mg Parthenolid, insbesondere 0,2- 0,6 mg, enthält.

10. Pflanzliches pharmazeutisches Präparat zur Behandlung von Migräne nach einem der vorangegangenen Ansprüche, **gekennzeichnet durch** einen Gehalt von 20- 100 mg Cimicifuga racemosa in Form der pflanzlichen Bestandteile und/ oder einer Zubereitung.

11. Pflanzliches pharmazeutisches Präparat zur Behandlung von Migräne nach einem der vorangegangenen Ansprüche, **gekennzeichnet durch** einen Gehalt von 20- 100 mg Vitex agnus castus in Form der pflanzlichen Bestandteile und/ oder einer Zubereitung, insbesondere 20- 40 mg.

12. Pflanzliches pharmazeutisches Präparat zur Behandlung von Migräne nach einem der vorangegangenen Ansprüche, **gekennzeichnet durch** einen Gehalt von 0,5-6 g Zingiber officinale in Form der pflanzlichen Bestandteile und/ oder einer Zubereitung, insbesondere 1-4 g.

13. Verwendung einer Zusammensetzung bestehend aus pflanzlichen Bestandteilen und/ oder Zubereitungen von Tanacetum parthenium in Kombination mit weiteren pflanzlichen Komponenten ausgewählt aus der Gruppe von Vitex agnus castus und/ oder Cimicifuga racemosa und/ oder Zingiber officinale zur Herstellung eines pflanzlichen pharmazeutischen Präparats für die Behandlung oder Prophylaxe von Migräne, insbesondere bei Frauen in Zusammenhang mit den Regelblutungen bzw. menstruellen Beschwerden oder bei zusätzlichen gastrointestinalen Beschwerden.

14. Verwendung einer Zusammensetzung nach Anspruch 13 zur Herstellung eines pflanzlichen pharmazeutischen Präparats, **gekennzeichnet durch** Tanacetum parthenium kombiniert mit Vitex agnus castus, Tanacetum parthenium kombiniert mit Cimicifuga racemosa, Tanacetum parthenium kombiniert mit Zingiber officinale und Tanacetum parthenium kombiniert mit Vitex agnus castus und Zingiber officinale als bevorzugte Kombinationspräparate.

## Claims

1. Herbal pharmaceutical preparation for the treatment of migraine, **characterised by** the combination of Tanacetum parthenium with other herbal ingredients selected from the Vitex agnus castus and/or Cimicifuga racemosa and/or Zingiber officinale groups.

2. Herbal pharmaceutical preparation for the treatment of migraine in accordance with Claim 1 **characterised by** the use of components and / or formulations of the pharmaceutically active ingredients.

3. Herbal pharmaceutical preparation for the treatment of migraine in accordance with Claim 1 or 2 **characterised by** formulations in the form of extracts, powders, distillates, infusion, tinctures and oils.

4. Herbal pharmaceutical preparation for the treatment of migraine in accordance with one of the above claims **characterised by** the combination of Tanacetum parthenium with Vitex agnus castus.

5. Herbal pharmaceutical preparation for the treatment of migraine in accordance with one of the above claims **characterised by** the combination of Tanacetum parthenium with Cimicifuga racemosa.

6. Herbal pharmaceutical preparation for the treatment of migraine in accordance with one of the above claims **characterised by** the combination of Tanacetum parthenium with Vitex agnus castus and Zingiber officinale.

7. Herbal pharmaceutical preparation for the treatment of migraine in accordance with one of the above claims **characterised by** the combination of Tanacetum parthenium with Zingiber officinale.

8. Herbal pharmaceutical preparation for the treatment of migraine in accordance with one of the above claims **characterised by** capsules, film tablets, solutions, dragées, suppositories, effervescent tablets, chewing tablets and effervescent granules as forms of administration.

9. Herbal pharmaceutical preparation for the treatment of migraine in accordance with one of the above claims **characterised by** a Tanacetum parthenium content in the form of the herbal ingredients and / or a formulation which contains 0.1 - 1 mg of parthenolide, in particular 0.2 - 0.6 mg.

10. Herbal pharmaceutical preparation for the treatment of migraine in accordance with one of the above claims **characterised by** a 20 - 100 mg Cimicifuga racemosa content in the form of the herbal ingredients and / or a formulation.

11. Herbal pharmaceutical preparation for the treatment of migraine in accordance with one of the above claims **characterised by** a Vitex agnus castus content of 20- 100 mg in the form of the herbal ingredients and / or a formulation, in particular 20 - 40 mg.

12. Herbal pharmaceutical preparation for the treatment of migraine in accordance with one of the above claims **characterised by** a Zingiber officinale content of 0.5 - 6 g in the form of the herbal ingredients and / or formulation, in particular 1 - 4 g.

13. Use of a combination of herbal ingredients and / or formulations of Tanacetum parthenium in combination with other herbal ingredients selected from the Vitex agnus castus and / or Cimicifuga racemosa and / or Zingiber officinale groups for the treatment or prevention of migraine in particular in women in association with menstruation or menstrual pain or additional gastro-intestinal pain.

14. Use of a compound in accordance with Claim 13 for the production of a herbal pharmaceutical preparation **characterised by** Tanacetum parthenium combined with Vitex agnus castus, Tanacetum parthenium combined with Cimicifuga racemosa, Tanacetum parthenium combined with Zingiber officinale and Tanacetum parthenium combined with Vitex agnus castus and Zingiber officinale as preferred compound preparations.

## Revendications

1. Produit pharmaceutique végétal pour le traitement des migraines, **caractérisé par** la combinaison de Tanacetum parthenium avec d'autres composants végétaux sélectionnés dans le groupe des Vitex agnus castus et/ ou Cimicifuga racemosa et/ ou Zingiber officinale.

2. Produit pharmaceutique végétal pour le traitement des migraines selon la revendication 1, **caractérisé par** l'utilisation de composants et/ ou de préparations des composants pharmaceutiques actifs.

3. Produit pharmaceutique végétal pour le traitement des migraines selon la revendication 1 ou 2, carcactérisé par les préparations sous la forme d'extraits, de poudres, de distillats, d'infusions, de teintures et d'huiles.

4. Produit pharmaceutique végétal pour le traitement des migraines selon l'une des revendications précédentes, **caractérisé par** la combinaison de Tanacetum parthénium avec Vitex agnus castus.

5. Produit pharmaceutique végétal pour le traitement des migraines selon l'une des revendications précédentes, **caractérisé par** la combinaison de Tanacetum parthenium avec Cimicifuga racemosa.

6. Produit pharmaceutique végétal pour le traitement des migraines selon l'une des revendications précédentes, **caractérisé par** la combinaison de Tanacetum parthenium avec Vitex agnus castus et Zingiber officinale.

7. Produit pharmaceutique végétal pour le traitement des migraines selon l'une des revendications précédentes, **caractérisé par** la combinaison de Tanacetum parthenium avec Zingiber officinale.

8. Produit pharmaceutique végétal pour le traitement des migraines selon l'une des revendications précédentes, **caractérisé par** des capsules, des comprimés recouverts d'une pellicule, des solutions, des dragées, des suppositoires, des comprimés effervescents, des comprimés à mâcher et des granulats effervescents en tant que formes d'administration.

9. Produit pharmaceutique végétal pour le traitement des migraines selon l'une des revendications précédentes, **caractérisé par** une teneur en Tanacetum parthenium sous la forme des composants végétaux et/ ou d'une préparation, qui contient 0,1 - 1 mg de parthenolide, en particulier 0,2 - 0,6 mg.

10. Produit pharmaceutique végétal pour le traitement des migraines selon l'une des revendications précédentes, **caractérisé par** une teneur de 20 - 100 mg de Cimicifuga racemosa sous la forme des composants végétaux et/ ou d'une préparation.

11. Produit pharmaceutique végétal pour le traitement des migraines selon l'une des revendications précédentes, **caractérisé par** une teneur de 20 - 100 mg de Vitus agnus castus sous la forme des composants végétaux et/ ou d'une préparation, en particulier 20 - 40 mg.

12. Produit pharmaceutique végétal pour le traitement des migraines selon l'une des revendications précédentes, **caractérisé par** une teneur de 0.5 - 6 g de Zingiber officinale sous la forme des composants végétaux et/ ou d'une préparation, en particulier 1 - 4 g.

13. Utilisation d'une composition comprenant des composants végétaux et/ ou des préparations de Tanacetum parthenium en combinaison avec d'autres composantes végétales sélectionnées dans le groupe Vitex agnus castus et/ ou Cimicifuga racemosa et/ ou Zingiber officinale pour la fabrication d'un produit pharmaceutique végétal pour le traitement ou la prophylaxie des migraines, en particulier chez la femme, en lien avec les menstruations ou les douleurs menstruelles ou en cas de douleurs gastro-intestinales supplémentaires.

14. Utilisation d'une composition selon la revendication 13 pour la fabrication d'un produit pharmaceutique végétal, **caractérisé par** Tanacetum parthenium combiné à Vitex agnus castus, Tanecetum parthenium combiné à Cimicifuga racemosa, Tanecetum parthenium combiné à Zingiber officinale et Tanecetum parthenium combiné à Vitex agnus castus et Zingiber officinale en tant que produits de combinaison favorisés.
